# EUROPÄISCHE PATENTSCHRIFT

(11) **EP 0 608 690 B1**
(45) Veröffentlichungstag und Bekanntmachung des Hinweises auf die Patenterteilung: **09.12.1998**
(21) Anmeldenummer: 94100211.5
(22) Anmeldetag: 08.01.1994
(51) Int. Cl.: C07D 207/267

(54) **Verfahren zur Herstellung von N-Alkenylpyrrolidonen**
Process for the preparation of N-alkenyl-pyrrolidones
Procédé pour la préparation de N-alkénylpyrrolidones

(30) Priorität: 28.01.1993 DE 4302325
(43) Veröffentlichungstag der Anmeldung: 03.08.1994
(73) Patentinhaber: BASF Aktiengesellschaft, 67063 Ludwigshafen (DE)
(72) Erfinder: Franz, Lothar, Dr., D-67112 Mutterstadt (DE); Huellmann, Michael, Dr., D-64646 Heppenheim (DE)

(56) Entgegenhaltungen:
- FR-A- 1 421 336
- FR-A- 2 103 021
- US-A- 2 669 570
- US-A- 3 526 620
- CHEMICAL ABSTRACTS, vol. 79, no. 11, 17. September 1973, Columbus, Ohio, US; abstract no. 66172v, Seite 433 ;
- CHEMISCHE BERICHTE Bd. 99, Nr. 7 , 1. Juli 1966 , WEINHEIM DE Seiten 2127 - 2135 H. BÖHME ET AL. 'Zur Kenntnis der N-[alpha-Alkoxyl-alkyl]-carbonsäureamide und der durch ihre thermische Spaltung entstehenden Enamide'

## Beschreibung

Die vorliegende Erfindung betrifft ein Verfahren zur Herstellung von N-Alkenylpyrrolidonen durch Umsetzung von N-Alkylpyrrolidonen bei erhöhten Temperaturen an sauren Heterogenkatalysatoren, ausgenommen Oxiden der Metalle der II., III., IV. und VI. Nebengruppe des Periodensystems der Elemente.

Aus der DE-A-21 35 211 ist ein Verfahren zur Herstellung von N-Vinylpyrrolidon durch Umsetzung von N-(2-Hydroxyethyl)-2-pyrrolidon an Katalysatoren von Oxiden der Metalle der II., III., IV. und VI. Nebengruppe des Periodensystems der Elemente unter atomosphärischem Druck bei Temperaturen von 250 bis 500°C in der Gasphase bekannt. Nachteil dieses Verfahrens ist die z.T. in beträchtlichem Umfang ablaufende Nebenreaktion zu 2-Pyrrolidon.

Nach US-A-2 669 570 kann N-Vinylpyrrolidon unter Verwendung von basischem oder neutralem Aluminiumoxid bei Temperaturen von 290 bis 335°C durch Wasserabspaltung in der Gasphase bei Drücken unterhalb von 100 mm Hg aus N-(2-Hydroxy-ethyl)pyrrolidon hergestellt werden. Ferner wird dort beschrieben, daß an brönstedsauren Kontakten, insbesondere an phosphorsauren Katalysatoren, nur unter erheblicher Zersetzung des Ausgangsmaterials die Eliminierung von Wasser aus N-Hydroxyethylpyrrolidonen in der Gasphase durchgeführt werden kann.

Die in Izv. Akad. SSSR, 901 bis 905 (1961) beschriebene unkatalysierte Essigsäurepyrolyse von N-(2-Acetoxyethyl)-pyrrolidon zeigt, daß unter diesen Bedingungen nur mäßige Ausbeuten erhalten werden.

Der vorliegenden Erfindung lag daher die Aufgabe zugrunde, den zuvor genannten Nachteilen abzuhelfen.

Demgemäß wurde ein neues und verbessertes Verfahren zur Herstellung von N-Alkenylpyrrolidonen der allgemeinen Formel I 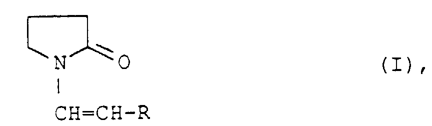 in der R Wasserstoff oder C₁- bis C₂₀-Alkyl bedeutet, durch Umsetzung von N-Alkylpyrrolidonen der allgemeinen Formel II 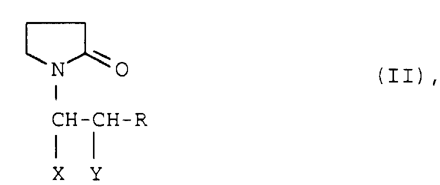 in der R die obengenannten Bedeutungen hat und X für Wasserstoff und Y für OH, C₁- bis C₂₀-Alkoxy oder C₂- bis C₂₀-Acyloxy stehen, bei Temperaturen von 200 bis 600°C und Drücken von 0,05 bis 5 bar, gefunden, welches dadurch gekennzeichnet ist, daß man in Gegenwart von phosphorhaltigen Verbindungen auf Trägern als saure Heterogenkatalysatoren arbeitet.

Das erfindungsgemäße Verfahren läßt sich wie folgt durchführen:

Die N-Alkylpyrrolidone II können bei Temperaturen von 200 bis 600°C, bevorzugt 250 bis 500°C, besonders bevorzugt 280 bis 480°C und Drücken von 0,05 bis 5 bar, bevorzugt 0,2 bis 2,5 bar, besonders bevorzugt 0,5 bis 1,5 bar mit phosphorhaltigen Verbindungen auf Trägern als saure Heterogenkatalysatoren, in Kontakt gebracht werden. In der Regel werden die N-Alkylpyrrolidone II im Dampfzustand über die Katalysatoren geleitet, wobei der Zusatz von inerten Gasen, wie Wasserstoff, Stickstoff und Wasserdampf, aber auch von inerten organischen Lösungsmitteln oder das Arbeiten unter vermindertem Druck vorteilhaft sein kann.

Als sauren Heterogenkatalysatoren eignen sich phosphorhaltige Verbindungen auf Trägern.

Als phosphorhaltige Verbindungen eignen sich Phosphorige Säuren wie Ortho-Phosphorigesäuren, Phosphorsäure wie Orthophosphorsäure, Pyrophosphorsäure, Metaphosphorsäure und kondensierte Phosphorsäuren wie Polyphosphorsäure, deren Anhydride und deren Salze der I., II., III., IV. und V. Hauptgruppe des Periodensystems der Elemente oder der I., II., III., V., VI., VII. und VIII. Nebengruppe des Periodensystems der Elemente oder der Lanthaniden wie Phosphate, Pyrophosphate, Monohydrogenphosphate und bevorzugt Dihydrogenphosphate, Alkyl- oder Arylester von Phosphorsäure oder Phosphoriger Säure oder alkyl- oder arylsubstituierte Phosphorsäure oder Phosphorige Säure auf inerten Trägern.

Als Salze seien aus der Gruppe Ia Lithium-, Natrium-, Kalium-, Rubidium-, Cäsium-, aus der Gruppe IIa Beryllium-, Magnesium-, Calcium-, Strontium-, Barium-, aus der Gruppe IIIa Bor-, Aluminium-, Gallium-, Indium-, Thallium-, aus der Gruppe IVa Titan-, Zirkon-, Hafnium-, aus der Gruppe Va Ammonium-, Antimon-, Wismut-, aus der Gruppe Ib Kupfer-, aus der Gruppe IIb Zink-, Cadmium-, Quecksilber-, aus der Gruppe IIIb Scandium-, Yttrium-, Lanthan-, aus der Gruppe Vb Vanadin-, Niob-, Tantal-, aus der Gruppe VIb Chrom-, aus der Gruppe VIIb Mangan-, aus der Gruppe VIII Eisen-, Cobalt-, Nickel-, aus der Lanthaniden Cer-, Praseodym-, Neodym-, Promethium-, Samarium-, Europium-, Gadolinium-, Terbium-, Dysprosium-, Holmium-, Erbium-, Thulium-, Ytterbium- und Lutetiumphosphat, -pyrophosphat, -monohydrogenphosphat und bevorzugt -dihydrogenphosphat genannt.

Als Alkyl- oder Arylester von Phosphorsäure oder Phosphoriger Säure eignen sich Mono-, Di- oder Triester mit 1 bis 8 Kohlenstoffatomen pro Alkylrest wie Methyl, Ethyl, n-Propyl, isopropyl, n-Butyl, iso-Butyl, sec.-Butyl, tert.-Butyl, n-Pentyl, iso-Pentyl, sec.-Pentyl, neo- Pentyl, 1,2-Dimethylpropyl und n-Hexyl oder Arylgruppen wie Phenyl, 1-Naphthyl, 2-Naphthyl, 1-Anthryl, 2-Anthtyl und 9-Anthryl, bevorzugt Phenyl, 1-Naphthyl und 2-Naphthyl, besonders bevorzugt Phenyl oder C₁- bis C₈-alkylsubstituiertes Phenyl, beispielsweise Triethylphosphat, Triethylphosphit, Phenylphosphat oder Phenylphosphit.

Als alkyl- oder arylsubstituierte Phosphorsäure oder Phosphorige Säure eignen sich als Substituenten C₁- bis C₈-Alkyl bevorzugt C₁- bis C₄-Alkyl wie Methyl, Ethyl, n-Propyl, iso-Propyl, n-Butyl, iso-Butyl, sec.-Butyl und tert.-Butyl oder Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl, beispielsweise Phenylphosphinsäure, Ethylphosphinsäure, Phenylphosphonsäure und Naphthylphosphonsäure.

Von den oben genannten phosphorhaltigen Verbindungen werden insbesondere die Dihydrogenphosphate, die Hydrogenphosphate der II. Hauptgruppe des Periodensystems der Elemente, die Phosphate des Vanadins und der Lanthaniden bevorzugt.

Als inerte Träger eignen sich beispielsweise Aluminiumoxide, Siliciumdioxid, Titandioxid und Zeolithe sowie deren Gemisch und Ton.

Die Substituenten R und Y in den Verbindungen I und II haben folgende Bedeutungen:
R
   - Wasserstoff oder
   - C₁- bis C₂₀-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl,
Y
   - Hydroxy,
   - C₁- bis C₂₀-Alkoxy, bevorzugt C₁- bis C₈-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy, besonders bevorzugt C₁- bis C₄-Alkoxy wie Methoxy, Ethoxy, n-Propoxy, iso-Propoxy, n-Butoxy, iso-Butoxy, sec.-Butoxy und tert.-Butoxy oder
   - C₂- bis C₂₀-Acyloxy, bevorzugt C₂- bis C₉-Acyloxy wie Methanoyloxy, Ethanoyloxy, n-Propanoyloxy, iso-Propanoyloxy, n-Butanoyloxy, iso-Butanoyloxy, sec.-Butanoyloxy, tert-Butanoyloxy, n-Pentanoyloxy, iso-Pentanoyloxy, sec.-Pentanoyloxy, neo-Pentanoyloxy, 1,2-Dimethylpentanoyloxy, n-Hexanoyloxy, iso-Hexanoyloxy, sec.-Hexanoyloxy, n-Heptanoyloxy-
   - iso-Heptanoyloxy, n-Octanoyloxy, iso-Octanoyloxy, besonders bevorzugt C₂- bis C₅-Acyloxy wie Methanoyloxy, Ethanoyloxy, n-Propanoyloxy, iso-Propanoyloxy, n-Butanoyloxy, iso-Butanoyloxy, sec.-Butanoyloxy und tert.-Butanoyloxy.

Ein besonderer Vorteil der Erfindung besteht in einer einfachen und wirtschaftlichen Herstellung der Katalysatoren sowie deren leichte Reaktivierung.

Die N-Alkenylpyrrolidone I eignen sich als Zwischenprodukte.

### Beispiele

### Beispiel 1

### Herstellung von mit Phosphorsäure getränktem SiO₂

330 g 75 %ige Phosphorsäure wird mit Wasser bis zu einem Gesamtvolumen von 860 ml aufgefüllt. Diese Lösung wird zu 490 g SiO₂ gegeben und die Suspension in einer Trommel 15 min gemischt. Nach der Filtration wird der Rückstand zunächst 64 h bei 120°C behandelt und anschließend 3 h bei 250°C calciniert.

### Beispiel 2

### Herstellung von Katalysator / La(H₂PO₄)₃

Zu 112,8 g Lanthannitrat x 6 H₂O gelöst in 300 ml Wasser wurden unter Rühren 168 g Diatomeenerde und 449,5 g einer 20 %igen wäßrigen Lösung von Ammoniumdihydrogenphosphat hinzugegeben. Die Lösung wurde erhitzt, das Wasser abgedampft, der Rückstand 3 h bei 120°C getrocknet und bei 3 h auf 400°C erhitzt.

### Beispiel 3

Als Reaktor wurde ein elektrisch beheizbares Quarzrohr von 150 cm Länge und 30 mm Innendurchmesser verwendet, dessen unterer Teil mit 225 ml Katalysator gefüllt war. Die Temperatur in dem Reaktor konnte mittels eines in deren Längsachse verschiebbaren Thermoelements gemessen werden. Der Kopf der Kolonne trug einen Tropftrichter für das Edukt II. Zur Kondensation des austretenden heißen Gasgemisches wurde das untere Ende des Ofens mit einem Kolben verbunden, dem ein Rückflußkühler aufgesetzt war. Um leicht flüchtige Anteile aus dem Gasstrom abzuscheiden, schloß sich eine mit Trockeneis-Aceton gekühlte Kühlfalle an. Durchführung, Umsätze und Selektivitäten sind Tabelle 1 zu entnehmen.

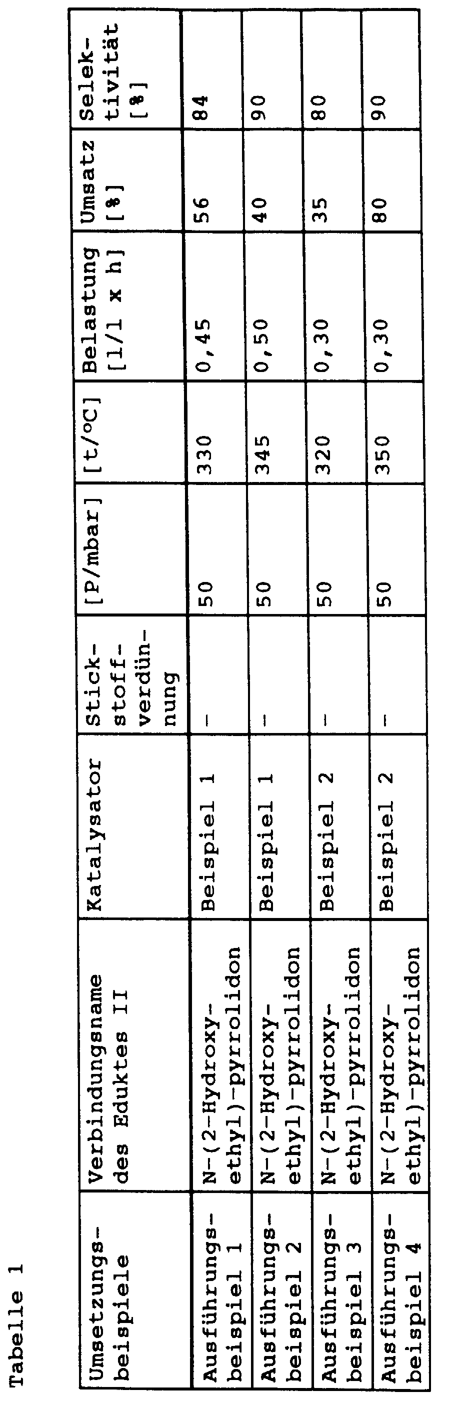

## Patentansprüche

1. Verfahren zur Herstellung von N-Alkenylpyrrolidonen der allgemeinen Formel I 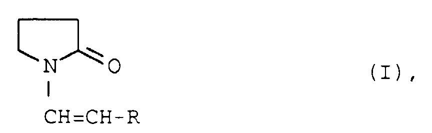 in der R Wasserstoff oder C₁- bis C₂₀-Alkyl bedeutet, durch Umsetzung von N-Alkylpyrrolidonen der allgemeinen Formel II 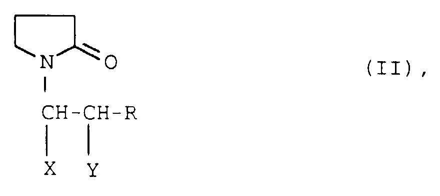 in der R die obengenannten Bedeutungen hat und X für Wasserstoff und Y für OH, C₁- bis C₂₀-Alkoxy oder C₂- bis C₂₀-Acyloxy stehen, bei Temperaturen von 200 bis 600°C und Drücken von 0,05 bis 5 bar in Gegenwart von sauren Heterogenkatalysatoren, dadurch gekennzeichnet, daß man als saure Heterogenkatalysatoren phosphorhaltige Verbindungen auf Trägern einsetzt.

2. Verfahren zur Herstellung von N-Alkenylpyrrolidonen I nach Anspruch 1, dadurch gekennzeichnet, daß man die Umsetzung in der Gasphase durchführt.

3. Verfahren zur Herstellung von N-Alkenylpyrrolidonen I nach Anspruch 1, dadurch gekennzeichnet, daß man die Umsetzung bei Temperaturen von 250 bis 500°C durchführt.

4. Verfahren zur Herstellung von N-Alkenylpyrrolidonen I nach Anspruch 1, dadurch gekennzeichnet, daß man die Umsetzung bei Temperaturen von 280 bis 480°C durchführt.

5. Verfahren zur Herstellung von N-Alkenylpyrrolidonen I nach Anspruch 1, dadurch gekennzeichnet, daß man die Umsetzung an Festbett- oder Wirbelkatalysatoren durchführt.

## Claims

1. A process for preparing N-alkenylpyrrolidones of the general formula I 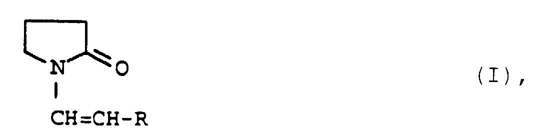 in which R is hydrogen or C₁-C₂₀-alkyl, by reacting N-alkylpyrrolidones of the general formula II 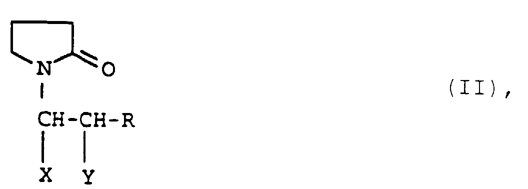 in which R has the abovementioned meanings, and X is hydrogen and Y is OH, C₁-C₂₀-alkoxy or C₂-C₂₀-acyloxy, at temperatures from 200 to 600°C under pressures from 0.05 to 5 bar in the presence of acidic heterogeneous catalysts, wherein phosphorus-containing compounds on carriers are employed as acidic heterogeneous catalysts.

2. A process for preparing N-alkenylpyrrolidones I as claimed in claim 1, wherein the reaction is carried out in the gas phase.

3. A process for preparing N-alkenylpyrrolidones I as claimed in claim 1, wherein the reaction is carried out at temperatures from 250 to 500°C.

4. A process for preparing N-alkenylpyrrolidones I as claimed in claim 1, wherein the reaction is carried out at temperatures from 280 to 480°C.

5. A process for preparing N-alkenylpyrrolidones I as claimed in claim 1, wherein the reaction is carried out on fixed bed or fluidized bed catalysts.

## Revendications

1. Procédé de préparation de N-alcénylpyrrolidones de formule générale I 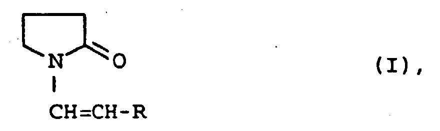 dans laquelle R représente un atome d'hydrogène ou un groupement alkyle en C₁-C₂₀, par réaction de N-alkylpyrrolidones de formule générale II 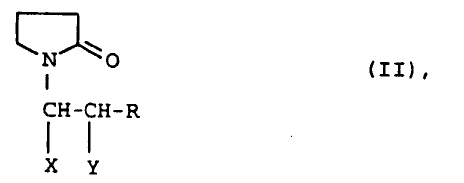 dans laquelle R prend la signification susmentionnée et X est mis pour un atome d'hydrogène et Y pour un groupement OH, alcoxy en C₁-C₂₀ ou acyloxy en C₂-C₂₀, à des températures de 200-600°C et sous des pressions de 0,05-5 bar en présence de catalyseurs hétérogènes acides, caractérisé en ce que l'on utilise des composés du phosphore sur supports, en tant que catalyseurs hétérogènes acides.

2. Procédé de préparation de N-alcénylpyrrolidones selon la revendication 1, caractérisé en ce que l'on mène la réaction en phase gazeuse.

3. Procédé de préparation de N-alcénylpyrrolidones I selon la revendication 1, caractérisé en ce que l'on mène la réaction à des températures de 250-500°C.

4. Procédé de préparation de N-alcénylpyrrolidones I selon la revendication 1, caractérisé en ce que l'on mène la réaction à des températures de 280-480°C.

5. Procédé de préparation de N-alcénylpyrrolidones I selon la revendication 1, caractérisé en ce que l'on mène la réaction sur des catalyseurs à lit fixe ou à lit fluidisé.
